(19) Europäisches Patentamt
European Patent Office
Office européen des brevets

(11) Veröffentlichungsnummer: **0 202 673**
**B1**

(12) **EUROPÄISCHE PATENTSCHRIFT**

(45) Veröffentlichungstag der Patentschrift: **29.08.90**

(51) Int. Cl.[5]: **C 07 F 17/00,** A 61 K 31/28

(21) Anmeldenummer: **86106912.8**

(22) Anmeldetag: **21.05.86**

# (54) Cytostatisch wirksame Titanocen-Komplexe.

(30) Priorität: **22.05.85 DE 3518447**

(43) Veröffentlichungstag der Anmeldung:
**26.11.86 Patentblatt 86/48**

(45) Bekanntmachung des Hinweises auf die Patenterteilung:
**29.08.90 Patentblatt 90/35**

(84) Benannte Vertragsstaaten:
**AT BE CH DE FR GB IT LI LU NL SE**

(56) Entgegenhaltungen:
**DE-A-2 923 334**

**EUROPEAN JOURNAL OF MEDICINAL CHEMISTRY, CHIMIE THERAPEUTIQUE, Band 19, Nr. 4, 1984, Seiten 347-352; P. KÖPF-MAIER et al.: "Tumorhemmung durch Metallocene: Titan-Komplexe des Typs TiCp2XYr und TiCpX2Yr**

**INORGANICA CHIMICA ACTA, Band 108, 1985, Seiten 99-103; P. KÖPF-MAIER et al.: "New antitumor titanocene derivatives containing hydrophilic ligands"**

**Dictionary of Organometallic Compounds Vol. 2, Seite 2258**

(73) Patentinhaber: **Köpf-Maier, Petra, Prof. Dr.**
**Bundesring 33**
**D-1000 Berlin 42 (DE)**

(73) Patentinhaber: **Köpf, Hartmut, Prof. Dr.**
**Bundesring 33**
**D-1000 Berlin 42 (DE)**

(72) Erfinder: **Köpf-Maier, Petra, Prof. Dr.**
**Bundesring 33**
**D-1000 Berlin 42 (DE)**
Erfinder: **Köpf, Hartmut, Prof. Dr.**
**Bundesring 33**
**D-1000 Berlin 42 (DE)**

(74) Vertreter: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40 (DE)**

(56) References cited:
**P.W. Atkins "Physical Chemistry" Oxford University Press ISBN 0 19 855148 7; 1978 Seiten 363 - 364**

**F. Seel "Grundlagen der analytischen Chemie", Seiten 126 und 134**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 202 673 B1**

**Beschreibung**

Die Erfindung betrifft Titanocen-Komplexe, die chemotherapeutisch wirksam sind und insbesondere cytostatische Eigenschaften aufweisen.

Cytostatisch wirksame Titanocen-Komplexe und diese Komplexe enthaltende Arzneipräparate wurden von den Erfindern in der DE—PS 29 23 334 und in European Journal of Medicinal Chemistry, Chimie Therapeutique, Band 19, Nr. 4, 1984, Seiten 347—352, beschrieben. Bei weiteren Untersuchungen wurden nun Titanocen-Komplexe gefunden, die einen höheren therapeutischen Index (Quotient aus toxizität und Aktivität) aufweisen und damit sicherer in der therapeutischen Anwendung sind.

Die Titanocen-Komplexe haben die allgemeine Formel I

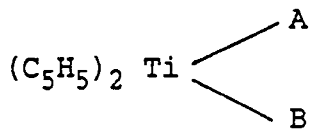

(I)

in der A die Grupe

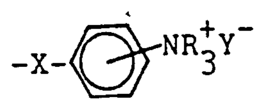

bedeutet, wobei X ein Sauerstoff- oder Schwefelatom ist, R Wasserstoff oder eine $C_{1-4}$-Alkylgruppe ist und Y ein Halogenid ist, oder A die Gruppe —O—CO—R′ bedeutet, wobei R′ die Gruppe $—CF_3$, $—CCl_3$, $—CBr_3$ $—CHCl_2$, $—CH_2Cl$, —CH=CH—COOH oder $—(CH_2)_nCOOH$ ist und n den Wert 0, 1, 2, 3 oder 4 hat, und B ein Halogenid ist oder wie der Rest A definiert ist.

X ist ein Sauerstoff- oder Schwefelatom, vorzugsweise ein Schwefelatom. Der Substituent $—NR_3^+Y^-$ kann in der o-, m- oder p-Stellung des Phenylringes stehen. Die Reste R haben gleiche oder unterschiedliche Bedeutung und sind ein Wasserstoffatom oder eine $C_{1-4}$-Alkylgruppe, wie z.B. Methyl, Ethyl, n-Propyl, Isopropyl, n-Butyl, tert.-Butyl oder Isobutyl. Y ist z.B. ein Fluorid-, Chlorid-, Bromid- oder Jodid-Anion. B kann eines dieser Halogenid-Anionen sein oder die vorstehende genannte Bedeutung für den Rest A haben.

Repräsentive Beispiele für Titanocen-Komplexe der allgemeinen Formel I sind:

(1) Bis(p-aminothiophenolato)bis($\eta^5$-cyclopentadienyl)titan(IV)-dihydrochlorid
(2) p-Aminothiophenolato-chloro-bis($\eta^5$-cyclopentadienyl)titan(IV)-hydrochlorid
(3) Bis(hydrogenmaleinato)bis($\eta^5$-cyclopentadienyl)titan(IV)
(4) Bis(trichloracetato)bis($\eta^5$-cyclopentadienyl)titan(IV)
(5) Bis-(p-methylaminothiophenolato)bis($\eta^5$-cyclopentadienyl)titan(IV)-dihydrojodid
(6) Bis(p-ethylaminothiophenolato)bis($\eta^5$-cyclopentadienyl)titan(IV)-dihydrojodid
(7) Bis(m-diethylaminophenolato)bis($\eta^5$-cyclopentadienyl)titan(IV)-hydrobromid
(8) o-Isopropylaminothiophenolato-jodo-bis($\eta^5$-cyclopentadienyl)titan(IV)-hydrojodid
(9) Trifluoracetato-fluoro-bis($\eta^5$-cyclopentadienyl)titan(IV)
(10) Hydrogensuccinato-bromo-bis($\eta^5$-cyclopentadienyl)titan(IV)

Diese Titanocen-Komplexe sind zum Teil bekannt und können nach Literaturvorschriften hergestellt werden.

Titanocen-Komplexe der allgemeinen Formel I, bei denen A die Gruppe

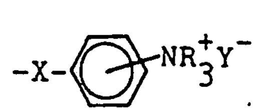

bedeutet, wobei X ein Sauerstoff- oder Schwefelatom ist, R Wasserstoff oder eine $C_{1-4}$-Alkylgruppe ist und Y ein Halogenid ist, und B ein Halogenid ist oder wie der Rest A definiert ist, sind neue Verbindungen.

Diese neuen Titanocen-Komplexe können dadurch hergestellt werden, daß man ein Titanocen-dihalogenid der allgemeinen Formel II

$$(C_5H_5)_2TiY_2 \qquad (II)$$

in der Y die vorstehende Bedeutung hat,

(a) mit einer Aminophenol- oder Aminothiophenol-Verbindung der allgemeinen Formel III

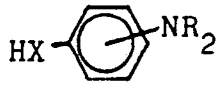

(III)

in der X und R die vorstehende Bedeutung haben, zu einem Titanocen-Komplex der allgemeinen Formel I umsetzt oder

(b) mit einem Lithium-aminophenolat oder -aminothiophenolat der allgemeinen Formel IV

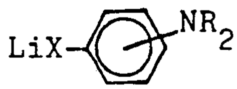

(IV)

in der X und R die vorstehende Bedeutung haben, zu einem Titanocen-Komplex der allgemeinen Formel I'

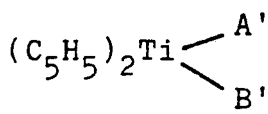

(I')

in der A' die Gruppe

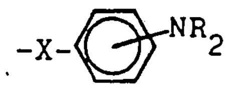

bedeutet und B' ein Halogenid ist oder wie der Rest A' definiert ist, umsetzt und den erhaltenen Titanocen-Komplex der allgemeinen Formel I' durch Umsetzen mit einer Halogenverbindung der allgemeinen Formel V

RY (V)

in der R und Y die vorstehende Bedeutung haben, in einen Titanocen-Komplex der allgemeinen Formel I überführt.

Zur Herstellung von Titanocen-Komplexen der allgemeinen Formel I, bei denen B ein Halogenid ist, wird ein Molverhältnis der Ausgangsverbindungen (II:III bei Verfahren (a); II:IV und I':V bei Verfahren (b)) von etwa 1:1 angewandt.

Zur Herstellung von Titanocen-Komplexen der allgemeinen Formel I, bei denen B wie der Rest A definiert ist, wird ein Molverhältnis der Ausgangsverbindungen (II:III bei Verfahren (a); II:IV und I':V bei Verfahren (b)) von etwa 1:2 angewandt.

Die Reaktionen werden in geeigneten Lösungsmitteln durchgeführt, z.B. in Wasser oder organischen Lösungsmitteln, wie Benzol, Hexan oder Tetrachlorkohlenstoff, oder Mischungen davon. Im Verfahren (b) kann das Zwischenprodukt der Formel I' isoliert werden oder aber ohne Isolierung direkt weiter zu dem Titanocen-Komplex der allgemeinen Formel I umgesetzt werden.

Im folgenden ist die Herstellung von Titanocen-Komplexen näher erläutert.

Beispiel 1

Herstellung von Bis(p-aminothiophenolato)bis($\eta^5$-cyclopentadienyl)titan(IV)-dihydrochlorid (Verbindung 1)

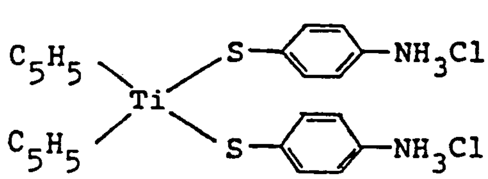

Zu einer Suspension von 1,25 g (5 mmol) Titanocen-dichlorid in 10 ml Tetrachlorkohlenstoff werden innerhalb 30 Minuten 1,25 g (10 mmol) p-Aminothiophenol in 40 ml Tetrachlorkohlenstoff getropft. Nach 60 h Rühren bei Raumtemperatur wird die tiefrote Verbindung abgesaugt und mit reichlich Tetrachlorkohlenstoff ausgewaschen. Durch Trocknen im Vakuum erhält man 2,3 g (Ausbeute 91%) feinkristalline Verbindung 1, F.>135°C Zersetzung.

$C_{22}H_{24}Cl_2N_2S_2Ti$ (MG 499,38)

| | C | H | Cl | N | S |
|---|---|---|---|---|---|
| ber. | 52,92 | 4,84 | 14,20 | 5,61 | 12,84% |
| gef. | 52,77 | 4,85 | 14,56 | 5,55 | 23,68% |

Beispiel 2

Herstellung von p-Aminothiophenolato-chloro-bis($\eta^5$-cyclopentadienyl)titan(IV)-hydrochlorid (Verbindung 2)

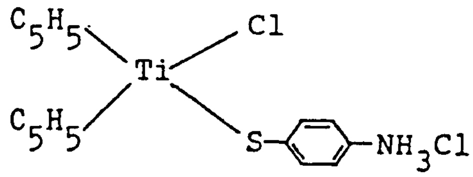

Zu einer Suspension von 2,5 g (10 mmol) Titanocen-dichlorid in 30 ml Tetrachlorkohlenstoff werden innerhalb 1,5 h 1,25 g (10 mmol) p-Aminothiophenol in 50 ml Tetrachlorkohlenstoff getropft. Nach 60 h Rühren bei Raumtemperatur wird die blaßviolette Verbindung abgesaugt und mit reichlich Tetrachlorkohlenstoff ausgewaschen. Durch Trocknen im Vakuum erhält man 3,4 g (Ausbeute 91%) der feinkristallinen Verbindung 2, F.>115°C (Zersetzung).

$C_{16}H_{17}Cl_2NSTi$ (MG 364,19)

| | C | H | N |
|---|---|---|---|
| ber. | 51,36 | 4,58 | 3,74% |
| gef. | 50,97 | 4,52 | 3,68% |

Beispiel 3

Herstellung von Bis(hydrogenmaleinato)bis($\eta^5$-cyclopentadienyl)titan(IV) (Verbindung 3)

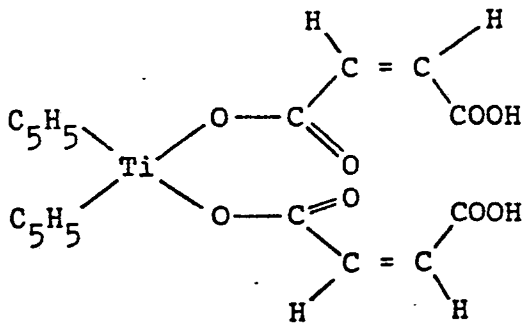

Zu einer Lösung von 0,5 g (2 mmol) Titanocen-dichlorid in 30 ml heißem Wasser werden 8 ml kaltgesättigte wäßrige Maleinsäurelösung gegeben. Beim Erkalten kristallisiert die Verbindung 3 in Form weinroter makroskopischer Kristalle. Durch Abpressen zwischen Filterpapier werden 0,58 g (Ausbeute 71%) analysenreine Verbindung 3 erhalten, F.>200°C (Zersetzung unter Braunfärbung).

$C_{18}H_{16}O_8Ti$ (MG 408,22)

| | C | H |
|---|---|---|
| ber. | 52,96 | 3,95% |
| gef. | 52,98 | 3,99% |

Literatur: K. Döppert, R. Sanchez-Delgado, H.—P. Klein und U. Thewalt, J. Organomet. Chem., 1982, *233*, 205.

Beispiel 4

Herstellung von Bis(trichloracetato)bis($\eta^5$-cyclopentadienyl)titan(IV) (Verbindung 4)

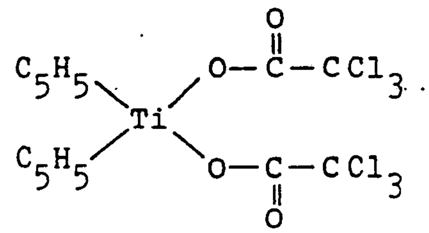

Zu einer Lösung von 0,5 g (2 mmol) Titanocen-dichlorid in 30 ml heißem Wasser werden 3,3 g (20 mmol) Trichloressigsäure gegeben, wobei die orangefarbene Verbindung 4 sofort ausfällt. Nach dem Erkalten wird abgesaugt und mit Wasser gewaschen. Durch Trocknen im Vakuum erhält man 0,88 g (Ausbeute 88%) der Verbindung 4, F. 173 bis 174°C.

$C_{14}H_{10}Cl_6O_4Ti$ (MG 502,78)

| | C | H |
|---|---|---|
| ber. | 33,44 | 1,99% |
| gef. | 33,70 | 2,00% |

Literatur: K. Döppert, Makromol. Chem., Rapid Commun., 1980, *1*, 519.

Beispiel 5

Herstellung von Bis(p-methylaminothiophenolato)bis($\eta^5$-cyclopentadienyl)titan(IV)-dihydrojodid (Verbindung 5)

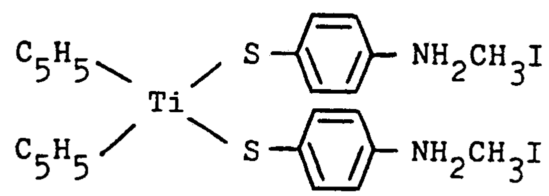

2,51 g (20 mmol) p-Aminothiophenol werden in 75 ml Benzol gelöst und durch Zutropfen der äquimolaren Menge (12,5 ml; 20 mmol) einer n-Butyllithium-Lösung in Hexan (c = 1,6 mol/l) in das Lithium-Salz $LiSC_6H_4$-p-$NH_2$ überführt und 1 h bei 25°C gerührt. Nach Zusatz von 2,50 g (10 mmol) $(C_5H_5)_2TiCl_2$ wird 12 h nachgerührt und anschließend die stöchiometrische Menge (1,25 ml; 20 mmol) $CH_3I$ (d = 2,28 g/ml) zugetropft. Nach weiteren 12 h Rühren bei Raumtemperatur wird die Reaktionslösung im Vakuum zur Trockene eingeengt und der verbleibenden Rückstand 8 h heiß mit 78 ml $CH_2Cl_2$ extrahiert. Aus der Extraktionslösung wird nach Einengen im Vakuum auf 20 ml und Kühlung bei −20°C das ausgefallene Produkt durch Filtration isoliert und 24 h im Vakuum getrocknet.

Ausbeute 4,83 g (68%) feinkristallines Produkt.

Farbe: schwarz (Feststoff), violett (in Lösung).

Analyse: $C_{24}H_{28}I_2N_2S_2Ti$

| | C | H | N |
|---|---|---|---|
| ber. | 40,58 | 3,97 | 3,94% |
| gef. | 40,26 | 4,23 | 4,28% |

$^1$H-NMR ($CDCl_3$; δ in ppm): 7,20—6,50m(8), 6,35s(10), 3,55s,br(4), 2,42s(6).

IR (KBr/ν in $cm^{-1}$): 3300m,bar, 3090w, 3030w, 2930w, 1612sh, 1590s, 1490vs, 1430s, 1275s,br, 1175m, 1095m, 1015s, 820vs, 705m, 635m,br.

Beispiel 6

Herstellung von Bis(p-ethylaminothiophenolato)bis($\eta^5$-cyclopentadienyl)titan(IV)-dihydrojodid (Verbindung 6)

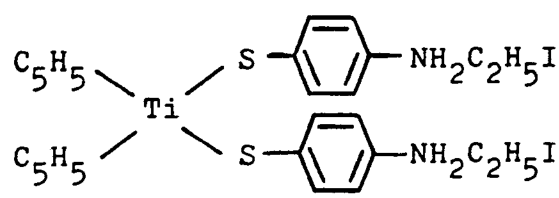

Die Darstellung erfolgt analog zu der von Verbindung 5 in Beispiel 5 unter Verwendung von 1,62 ml (20 mmol) $C_2H_5I$ (d = 1,93 g/ml) anstelle von $Ch_3I$.

Ausbeute 3,98 g (54%) feinkristallines Produkt.

Farbe: schwarz (Feststoff), violett (in Lösung).

Analyse: $C_{26}H_{32}I_2N_2S_2Ti$

| | C | H | N |
|---|---|---|---|
| ber. | 42,29 | 4,38 | 3,80% |
| gef. | 42,13 | 4,38 | 3,75% |

$^1$H-NMR ($CDCl_3$; δ in ppm): 6,77—6,40m(8), 6,24s(10,) 3,52s,br(4), 2,69q(4), 1,14t(6).

IR (KBr/ν in $cm^{-1}$): 3380m,br, 2810w, 1678m, 1618s, 1590s, 1485s, 1435m, 1275s,br, 1175m, 1015m, 820vs, 690m,br.

Die erfindungsgemäßen Titanocen-Komplexe zeigen cytostatische Wirkung und eignen sich insbesondere zur Behandlung von soliden Tumoren, z.B. Tumoren des Digestionstraktes, Lungen- und Mamma-Carcinoma.

Aktivität gegenüber Erhlich-Aszites-Tumor

Weibliche $CF_1$-Mäuse erhalten mittels intraperitonealer Injektion je etwa $6 \cdot 10^6$ Erhlich-Aszites-Tumorzellen und 24 Stunden später eine einmalige intraperitoneale Substanzgabe (Dosisbereich 20 bis 500 mg/kg) in physiologischer Kochsalzlösung (0,4 ml). Es werden jeweils 5 bis 10 Tiere pro Dosis getestet. Gegebenenfalls kann auf einen pH-Wrt von 4 bis 7 gepuffert werden, z.B. mit Natriumhydrogencarbonat oder Tris-(hydroxymethyl)-aminomethan, um eine lokale Irriation am Injektionsort zu vermeiden. In jeder Versuchsreihe wird eine Gruppe von unbehandelten Kontrolltieren, denen 0,4 ml physiologische Kochsalzlösung ohne Substanzgabe intraperitoneal injiziert wurde, mitgeführt.

Die Beurteilung der Tumorentwicklung in den einzelnen Dosisbereichen erfolgt anhand von Gewichtsverlauf und Überlebensdauer. Bei jeder Substanz werden die dosisabhängige Anzahl von Tumortodesfällen, Toxizitätstodesfällen und überlebenden, geheilten Tieren sowie die zugehörige prozentuale Zunahme der mittleren Überlebensdauer bestimmt.

Die bei der Testung der Verbindungen 1 bis 4 und von Titanocen-dichlorid als Vergleichssubtanz erzielten Ergebnisse sind in der folgenden Tabelle 1 wiedergegeben.

Tabelle 1

| Testverbindung | Optimale Heilungsrate | Therapeutischer Index ($LD_{50}/ED_{90}$) |
|---|---|---|
| Verbindung 1 | 100% | 4,3 |
| Verbindung 2 | 100% | 4,4 |
| Verbindung 3 | 100% | 3,8 |
| Verbindung 4 | 100% | 5,5 |
| Titanocen-dichlorid (Vergleich) | 90 - 100% | 3,3 |

Die Testergebnisse sind graphisch in den Fig. 1 bis 5 dargestellt. Aus ihnen ist ersichtlich, daß die erfindungsgemäß verwendeten Titanocen-Komplexe gegenüber Titanocendichlorid einen höheren therapeutischen Index (entsprechend einer größeren therapeutischen Breite) aufweisen. Sie sind damit sicherer in der therapeutischen Anwendung. Zudem zeigen die Verbindungen 1, 2 und 3 höhere Wasserlöslichkeit als Titanocen-dichlorid, wodurch ihre Applikation und Dosierung erleichtert wird.

Aktivität gegenüber aszitischem und solidem Sarkom 180

Das verwendete Sarkom 180 wurde von Deutschen Krebsforschungszentrum (Heidelberg, Deutschland) erhalten. Es wurde als intraperitoneal wachsende Tumorlinie auf weiblichen NMRI-Mäusen propagiert, die unter Standardbedingungen (Altromin-Futter und Leitungswasser ad libitum, 12 h Hell-Dunkel-Rhythmus) gehalten wurden.

Die Antitumortestung erfolgte gegen Sarkom 180 sowohl in fluider aszitischer als auch in solider subkutan wachsender Form. Für die Testung gegenüber aszitischem oder solidem Sarkom 180 wurde der aszitische Tumor von Donormäusen geerntet, mit Kochsalzlösung 1:3 (V:V) verdünnt und entweder in die Bauchhöhle oder subkutan in den Achselbereich von weiblichen NMRI-Mäusen injiziert. Jedes Tier erhielt etwa $10^7$ Tumorzellen. Der Tag der Tumortransplantation wurde als Tag 0 des Experiments definiert.

Vor Verabfolgung der Testverbindungen wurden den angewandten Dosen entsprechende Proben in

DMSO gelöst, mit dem 9fachen Volumen Kochsalzlösung aufgefüllt und den Mäusen in Volumina von 0,02 ml pro g Körpergewicht appliziert. Die verschiedenen Dosen wurden entweder als einzige Injektion am Tag 1 oder als 3fache Injektion an den Tagen 1, 3 und 5 verabfolgt. Einzelheiten über der die Dosen und die Tierverteilung sind in den Tabellen 2 und 3 genannt. Die unbehandelten tumortragenden Kontrolltiere erhielten nur Injektionen des DMSO/Kochsalz-Gemisches (0,02 ml/g).

Jeden Tag wurden die auftretenden Todesfälle aufgezeichnet. Innerhalb von 8 Tagen nach der Tumortransplantation auftretende Todesfälle wurden als Toxizitätstodesfälle aufgrund von Substanztoxizität definiert. Die ausgewerteten Parameter waren im Falle von aszitischem Sarkom 180 die mittlere Überlebenszeit (MST) und die Erhöhung der Lebensdauer (ILS), wobei sich letztere aus der Beziehung der mittleren Überlebenszeit einer behandelten Gruppe zu der der entsprechenden Kontrollgruppe, ausgedrückt als Prozentsatz und substrahiert mit 100%, errechnet. Der Stichtag zur Auswertung des Experiments und zur Bestimmung der Zahl an überlebenden (geheilten) Tieren war Tag 80. Im Falle von solidem Sarkom 180 wurden die Tumoren am Tag 9 nach der Transplantation entfernt und mit einer Genauigkeit von ±1 mg gewogen. Aus den Tumorgewichten der behandelten Tiere und Kontrolltiere wurden die T/C-Werte folgendermaßen errechnet:

$$T/C\ (\%) = \frac{\text{mittleres Tumorgewicht einer Dosisgruppe} \times 100}{\text{mittleres Tumorgewicht der Kontrollgruppe}}$$

Es wurden folgende Ergebnisse erhalten:

Tabelle 2

Aktivität gegenüber solidem Sarkom 180

| Testverbindung | Dosis (mg/kg) | Toxizitätstodesfälle/behandelte Tiere | Tumorgewicht[a] (g) | T/C (%) |
|---|---|---|---|---|
| Titanocen-dichlorid | 1 x 40 | -/5 | 1,31 ± 0,24 | 111 |
| | 1 x 50 | -/5 | 0,78 ± 0,34 | 66 |
| | 3 x 40 | -/5 | 0,75 ± 0,32 | 58 |
| | 3 x 50 | -/5 | 0,29 ± 0,14 | 23 |
| Titanocen-dibromid | 1 x 50 | -/5 | 1,27 ± 0,37 | 108 |
| | 1 x 60 | -/5 | 1,21 ± 0,37 | 94 |
| | 3 x 50 | -/5 | 0,69 ± 0,17 | 53 |
| | 3 x 60 | 1/5 | 0,48 ± 0,10 | 37 |
| Verbindung 3 | 1 x 80 | -/5 | 1,13 ± 0,18 | 95 |
| | 1 x 120 | -/5 | 0,87 ± 0,41 | 73 |
| | 3 x 60 | -/5 | 0,91 ± 0,25 | 76 |
| | 3 x 100 | -/5 | 0,63 ± 0,14 | 53 |

Tabelle 2 (Fortsetzung)

| Testverbindung | Dosis (mg/kg) | Toxizitätstodesfälle/behandelte Tiere | Tumorgewicht[a] (g) | T/C (%) |
|---|---|---|---|---|
| Verbindung 4 | 1 x 220 | -/5 | 1,14 ± 0,26 | 96 |
| | 1 x 320 | -/5 | 0,79 ± 0,28 | 67 |
| | 3 x 200 | -/5 | 1,26 ± 0,30 | 106 |
| | 3 x 300 | 1/5 | 0,44 ± 0,14 | 37 |
| Verbindung 1 | 1 x 60 | -/5 | 1,30 ± 0,30 | 109 |
| | 1 x 100 | -/5 | 1,03 ± 0,26 | 86 |
| | 3 x 60 | -/5 | 0,47 ± 0,12 | 39 |

(a) Angegeben sind Durchschnittswerte und Standardabweichungen

Tabelle 3

Aktivität gegenüber aszitischem Sarkom 180

| Testverbindung | Dosis (mg/kg) | Toxizitätstodesfälle/behandelte Tiere | MST[b] (Tage) | ILS (%) | Überlebende Tiere/behandelte Tiere |
|---|---|---|---|---|---|
| Titanocendichlorid | 1 x 40 | -/6 | 27,3 (20-49) | 32 | -/6 |
| | 1 x 40 | -/5 | 32,0 (18-80) | 81 | 1/5 |
| | 1 x 50 | -/6 | 54,0 (25-80) | 161 | 3/6 |
| | 1 x 50 | -/5 | 50,0 (25-80) | 184 | 2/5 |
| Titanocendibromid | 1 x 50 | -/5 | 28,4 (22-34) | 61 | -/5 |
| | 1 x 60 | -/5 | 40,2 (20-80) | 128 | 1/5 |
| Verbindung 3 | 1 x 80 | -/5 | 32,2 (15-80) | 95 | 1/5 |
| | 1 x 120 | -/5 | 37,2 (21-80) | 125 | 1/5 |
| Verbindung 4 | 1 x 220 | -/5 | 17,2 (15-20) | 4 | -/5 |
| | 1 x 320 | -/5 | 32,4 (18-80) | 96 | 1/5 |
| Verbindung 1 | 1 x 60 | -/5 | 19,0 (15-25) | 15 | -/5 |
| | 1 x 100 | -/5 | 39,4 (19-80) | 139 | 1/5 |

(b) angegeben sind die Mittelwerte der Überlebenszeit, in Klammern der Bereich der Einzelwerte

Aktivität gegenüber B16-Melanom und Colon-38-Adenocarcinom

Das verwendete B-16-Melanom und Colon-38-Carcinom wurden vom NCI Liasion Office, Institut Jules Bordet, Brüssel, Belgien, erhalten und als solide, subkutan wachsende Tumorlinien auf männlichen C-57-BL/6J-Mäusen propagiert. Für die Testung wurden die Tumoren auf weibliche $B_6D_2F_1$-Mäuse transplantiert. Alle Tiese wurden unter Standardbedingungen (22—23°C, Leitungswasser und Altromin-Futter ad libitum, 12 h Hell-Dunkel-Rhythmus gehalten).

Für die Tumortransplantation am Tag 0 der Testreihe wurden solide Tumoren aus Donormäusen entfernt, mit der Schere zerkleinert und in kleine Teilchen überführt. Nach dem Suspendieren im zweifachen Volumen Hank-Salzlösung wurden jeweils 0,3 ml subkutan in dem Achselbereich geimpft, so daß einzelne solide Tumoren wuchsen.

Die Testverbindungen wurden entweder als Einzelinjektion am Tag 1 oder als 3fache Injektionen an den Tagen 1, 3 und 5 oder als 5fache Injektionen an den Tagen 1, 3, 5, 7 und 9 verabfolgt. Die angewandten Dosen und die Tierverteilung sind in Tabelle 4 genannt. Die in einer DMSO-Kochsalz-Mischung (1:9; V:V) gelösten oder suspendierten Substanzen wurden stets intraperitoneal injiziert. Die Konzentrationen wurden so gewählt, daß jede Maus ein Gesamtvolumen von 0,4 bis 0,5 ml erhielt. Kontrolltiere erhielten nur eine einzelne, 3fache oder 5fache Injektion des DMSO-Kochsalz-Gemisches (0,5 ml/Tier) ohne Wirkstoffzugabe.

Die Anzahl der Todesfälle wurde täglich augezeichnet. Todesfälle, die innerhalb von 7 Tagen (einzelne Injektion), 11 Tagen (3fache Injektion) oder 15 Tagen (5fache Injektion) nach der Tumortransplantation auftraten, wurden als Toxizitätstodesfälle aufgrund der Substanztoxizität definiert.

Im Falle des B16-Melanoms wurden die Tumoren am Tag 10 nach 1facher und 3facher Injektion bzw. am Tag 15 nach 5facher Injektion entfernt und gewogen. Die Colon-38-Carcinoma wurden alle am Tag 15 der Versuchreihe ausgewertet.

In der folgenden Tabelle 4 ist die Antitumoraktivität, ausgedrückt als T/C-Wert, angegeben.

Tabelle 4

Aktivität gegenüber B16-Melanom und Colon-38-Adenocarcinom

| Testverbindung | Dosis (mg/kg) | T/C (%) B16-Melanom | T/C (%) Colon-38-Carcinom |
|---|---|---|---|
| Titanocen-dichlorid | 1 x 50 | 61 | 41 |
| | 3 x 40 | 69 | 28 |
| | 3 x 50 | 37 | - |
| Titanocen-dibromid | 1 x 60 | 42 | 36 |
| | 3 x 50 | 48 | 51 |
| | 3 x 60 | 44 | - |
| Verbindung 1 | 1 x 100 | 80 | 61 |
| | 3 x 60 | 36 | 38 |
| Verbindung 3 | 1 x 120 | 89 | 72 |
| | 3 x 100 | 50 | 50 |
| Verbindung 4 | 1 x 320 | 85 | 48 |
| | 3 x 300 | 41 | - |

Aktivität gegenüber heterotransplantierten Human-Tumoren

Es wurde dieselbe Testmethodik wie bei der Testung gegenüber B16-Melanom und Colon-38-Carcinom angewandt. Die Testverbindungen wurden in äquivalenten und äquitoxischen Dosen, unterteilt in 5 Injektionen, verabfolgt. Hierbei erfolgte die erste Injektion beim Mammacarcinom am Tag 8, bei den Adenocarcinoma des Rektums und der Lunge am Tag 10 und beim kleinzelligen Lungencarcinom am Tag 26 nach der Tumortransplantation. Die T/C-Werte wurden 3 Tage nach der letzten Substanzinjektion

bestimmt, d.h. am Tag 23 beim Mammacarcinom, am Tag 25 bei den Andenocarcinoma des Rektums und der Lunge und am Tag 41 beim kleinzelligen Lungencarcinom.

Es wurden die in Tabelle 5 genannten Ergebnisse erhalten.

Tabelle 5

Aktivität gegenüber einigen heterotransplantierten Human-Tumoren

| Testverbindung | Dosis (mg/kg) | T/C (%) (bezogen auf das mittlere relative Tumorvolumen) | | | |
|---|---|---|---|---|---|
| | | Rektum-Adeno-carcinom | Lungen-Adeno-carcinom | Klein-zelliges Lungen-carcinom | Mamma-carcinom |
| Titanocen-dichlorid | 5 x 15 | 38 | 35 | 110 | 49 |
| Titanocen-dibromid | 5 x 20 | 48 | 53 | 96 | 44 |
| Verbindung 1 | 5 x 50 | 59 | 49 | 83 | 49 |
| Verbindung 3 | 5 x 30 | 89 | 35 | 65 | 40 |

Die bei der Testung an den Adenocarcinoma der Lunge und des Rektums erzielten Ergebnisse sind graphisch in den Fig. 6 und 7 dargestellt.

Zur Krebsbekämpfung können die erfindungsgemäßen Titanocen-Komplexe als solche oder als Arzneimittel eingesetzt werden, die mindestens einen Titanocen-Komplex der allgemeinen Formel I neben pharmazeutisch verträglichen Träger-, Verdünnungs- und/oder Hilfsstoffen enthälten. Die pharmazeutische Zubereitung der Wirkstoffe erfolgt vorzugsweise in Form von Einheitsdosen, die auf die jeweilige Applikationsart abgestimmt sind. Eine Einheitsdosis kann z.B. eine Tablette, eine Kapsel, ein Suppositorium oder eine abgemessene Volumenmenge eines Pulvers, eines Granulats oder eine Lösung oder Suspension sein. Unter "Einheitsdosis" wird eine physikalisch bestimmte Einheit verstanden, die eine individuelle Menge des Wirkstoffes in Mischung mit einem geeigneten pharmazeutischen Träger-, Verdünnungs- und/oder Hilfsstoff enthält. Dabei wird die Wirkstoffmenge so gewählt, daß eine oder mehrere Einheiten üblicherweise für eine einzelne therapeutische Verabreichung ausreichen. Die Einheitsdosis kann auch unterteilbar sein, z.B. in form von gekerbten Tabletten, wenn für eine einzelne therapeutische Verabreichung nur eine Bruchteil, z.B. eine Hälfte oder ein Viertel, der unterteilbaren Einheit benötigt wird. Die Arzneimittel der Erfindung enthalten, wenn sie in Einheitsdosis vorliegen, 1 bis 10 000 mg, vorzugsweise 5 bis 7 500 mg, Wirkstoff.

Die Arzneimittel der Erfindung werden vorzugsweise oral, rectal oder parenteral, z.B. intravenös, subcutan, intramuskulär, intrapleural, intraperitoneal, intrafokal oder perifokal, angewandt. Die therapeutische Verabreichung kann mittels Infusion kontinuierlich über mehrere Stunden oder durch ein- bis mehrmalige Einzelgaben oder Einzelinjektionen erfolgen. Die Verabreichungsfolge und die verabreichte Dosis können in Abhängigkeit von Natur und Stadium der Erkrankung sowie abhängig von Behandlungsregime, insbesondere von Anzahl und Dosierungshöhe verabreichter Kombinationspräparate, stark variieren. Beispielsweise kann eine Anfangsbehandlung mit täglich 200 bis 800 mg. i.v. oder mit Einzeldosen, z.B. 10 bis 40 mg/kg i.v., in entsprechenden Intervallen und eine darauffolgende Dauerbehandlung mit 1 bis 4 Tabletten zu je 50 mg Wirkstoff erfolgen.

Die Arzneimittel bestehen in der Regel aus den erfindungsgemäßen Wirkstoffen und nicht-toxischen, pharmazeutisch annehmbaren Arzneimittelträgern, die als Zumischung in fester, halbfester oder flüssiger Form oder als Umhüllungsmittel, z.B. in Form einer Kapsel, eines Tablettenüberzuges, eines Beutels oder eines anderen Behältnisses für den Wirkstoff in Anwendung kömmen. Der Träger kann hierbei z.B. als Vermittler für die Arzneimittelaufnahme durch den Körper, als Formulierungshilfsmittel, Süßungsmittel, Geschmacksmittel, Farbstoff oder Konservierungsmittel dienen.

Für die orale Verabreichung eignen sich z.B. Tabletten, Dragées, Hart- und Weichgelatinekapseln,

dispergierbare Pulver, Granulate, wässrige und ölige Suspensionen, Emulsionen, Lösungen und Sirupe.

Tabletten können inerte Verdünnungsmittel, wie Calciumcarbonat, Calciumphosphat, Natriumphosphat oder Lactose; Granulierungs- und Verteilungsmittel, wie Maisstärke oder Alginate; Bindemittel, wie Stärke, Gelatine oder Akaziengummi; und Gleitmittel, wie Aluminium- oder Magnesiumstearat, Talcum oder Siliconöl, enthalten. Gegebenenfalls sind die Tabletten mit einem Überzug versehen, der auch so beschaffen sein kann, daß er eine verzögerte Auflösung und Resorption des Arzneimittels im Gastrointestinaltrakt und damit z.B. eine bessere Verträglichkeit oder eine längere Wirkungsdauer bewirkt.

Gelatinekapseln können der Wirkstoff im Gemisch mit einem festen (z.B. Calciumcarbonat oder Kaolin) oder öligen (z.B. Oliven-, Erdnuß- oder Paraffinöl) Verdünnungsmittel enthalten.

Als Suspendiermittel eignen sich z.B. Natriumcarboxymethylcellulose, Methylcellulose, Hydroxypropylcellulose, Natriumalginat, Polyvinylpyrrolidon, Traganthgummi oder Akaziengummi; als Dispergier- und Benetzungsmittel z.B. Polyoxyethylenstearat, Heptadecaethylenoxycetanol, Polyoxyethylensorbitmonooleat, Polyoxyethylensorbitanmonooleat oder Lecithin; als Konservierungsmittel z.B. Methyl- oder Propylhydroxybenzoat; als Geschmacks- und Süßungsmittel z.B. Saccharose, Lactose, Dextrose oder Invertzuckersirup.

Ölige Suspensionen können z.B. Erdnüß-, Oliven-, Sesam-, Kokos- oder Paraffinöl sowie Verdickungsmittel, wie Bienenwachs, Hartparaffin oder Cetylalkohol, Sußungsmittel, Geschmacksmittel und/oder Antioxidantien enthalten.

In Wasser dispergierbare Pulver und Granulate enthalten den Wirkstoff im Gemisch mit Dispergier-, Benetzungs- und Suspendiermitteln, z.B. den vorstehend genannten Substanzen und/oder Dimethylsulfoxid, sowie mit Süßungsmitteln, Geschmacksmitteln und/oder Farbstoffen.

Emulsionen können z.B. Oliven-, Erdnuß- oder Paraffinöl neben Emulgatoren, wie Akaziengummi, Traganthgummi, Phosphatiden, Sorbitanmonooleat oder Polyoxyethylensorbitanmonooleat, Süßungsmittel und/oder Geschmacksmitteln enthalten.

Zur rectalen Anwendung eignen sich Suppositorien, die mit Hilfe von bei Rektaltemperatur schmelzenden Bindemitteln hergestellt werden, z.B. Kakaobutter oder Polyethylenglykolen.

Parenteral können die Arzneimittel als sterile isotonische Kochsalzlösungen oder sonstige Lösungen angewandt werden. Um eine gleichmäßige Lösung bzw. Suspendierung zu erzielen, kann ein Lösungsvermittler, wie Dimethylsulfoxid, zugesetzt werden, jedoch ist dies meist nicht erforderlich.

In allen Darreichungsformen können die Arnzeimittel der Erfindung außerdem Puffersubstanzen enthalten, z.B. Natriumhydrogencarbonat oder Tris(hydroxymethyl)aminomethan. Neben den erfindungsgemäßen Titanocen-Komplexen können die Arzneimittel einen oder mehrere andere pharmakologisch aktive Bestandteile aus anderen cytostatisch wirksamen Arnzeimittelgruppen enthalten, z.B. Alkylantien, Antimetaboliten sowie cytostatische Alkaloide, Antibiotika, Enzyme und Schwermetallverbindungen. Ferner können die Arzneimittel gegebenenfalls immunsuppressiv wirksame Substanzen und Vitamine enthalten. Die genannten Zusatzstoffe können auch in gesonderten pharmazeutischen Zubereitungen als Kombinationspräparate zu den erfindungsgemäßen Wirkstoffen gegeben werden.

Der Wirkstoffgehalt der Arnzeimittel beträgt gewöhnlich 0,01 bis 95 Gewichtsprozent, vorzugsweise 0,1 bis 85 Gewichtsprozent, bezogen auf das fertige Arzneimittel.

**Patentansprüche**

1. Titanocen-Komplexe der allgemeinen Formel I

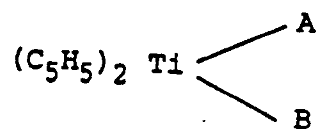

(I)

in der A die Gruppe

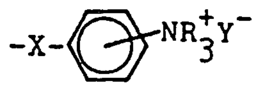

bedeutet, wobei X ein Sauerstoff- oder Schwefelatom ist, R Wasserstoff oder eine $C_{1-4}$-Alkylgruppe ist und Y ein Halogenid ist, oder A die Gruppe —O—CO—R' bedeutet, wobei R' die Gruppe $—CF_3$, $—CCl_3$, $—CBr_3$ $—CHCl_2$, $—CH_2Cl$, —CH=CH—COOH oder $—(CH_2)_nCOOH$ ist und n den Wert 0, 1, 2, 3 oder 4 hat, und B ein Halogenid ist oder wie der Rest A definiert ist;
zur Verwendung als Arzneistoffe.

2. Titanocen-Komplexe der allgemeinen Formel I

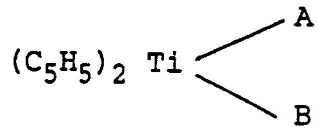

(I)

in der A die Gruppe

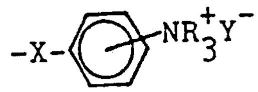

bedeutet, wobei X ein Sauerstoff- oder Schwefelatom ist, R Wasserstoff oder eine $C_{1-4}$-Alkylgruppe ist und Y ein Halogenid ist, und B ein Halogenid ist oder wie der Rest A definiert ist.

3. Titanocen-Komplexe nach Anspruch 1, ausgewählt unter:

Bis(p-aminothiophenolato)bis($\eta^5$-cyclopentadienyl)titan(IV)-dihydrochlorid
p-Aminothiophenolato-chloro-bis($\eta^5$-cyclopentadienyl)titan(IV)-hydrochlorid
Bis-(p-methylaminothiophenolato)bis($\eta^5$-cyclopentadienyl)titan(IV)-dihydrojodid
Bis(p-ethylaminothiophenolato)bis($\eta^5$-cyclopentadienyl)titan(IV)-dihydrojodid
Bis(m-diethylaminophenolato)bis($\eta^5$-cyclopentadienyl)titan(IV)-dihydrobromid
und
o-Isopropylaminothiophenolato-jodo-bis($\eta^5$-cyclopentadienyl)titan(IV)-hydrojodid.

4. Pharmazeutische Zusammensetzung, enthaltend eine wirksame Menge mindestens eines Titanocen-Komplexes der allgemeinen Formel I

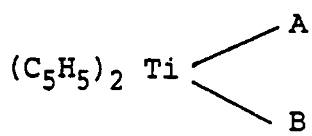

(I)

in der A die Gruppe

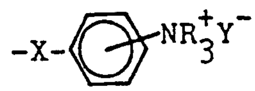

bedeutet, wobei X ein Sauerstoff- oder Schwefelatom ist, R Wasserstoff oder eine $C_{1-4}$-Alkylgruppe ist und Y ein Halogenid ist, oder A die Gruppe $—O—CO—R'$ bedeutet, wobei R' die Gruppe $—CF_3$, $—CCl_3$, $—CBr_3$ $—CHCl_2$, $—CH_2Cl$, $—CH{=}CH—COOH$ oder $—(CH_2)_nCOOH$ ist und n den Wert 0, 1, 2, 3 oder 4 hat, und B ein Halogenid ist oder wie der Rest A definiert ist;
und übliche Träger- oder Hilfsstoffe.

5. Pharmazeutische Zusammensetzung nach Anspruch 4, dadurch gekennzeichnet, daß sie mindestens einen Titanocen-Komplex ausgewählt unter
Bis(p-aminothiophenolato)bis($\eta^5$-cyclopentadienyl)titan(IV)-dihydrochlorid
p-Aminothiophenolato-chloro-bis($\eta^5$-cyclopentadienyl)titan(IV)-hydrochlorid
Bis-(p-methylaminothiophenolato)bis($\eta^5$-cyclopentadienyl)titan(IV)-dihydrojodid
Bis(p-ethylaminothiophenolato)bis($\eta^5$-cyclopentadienyl)titan(IV)-dihydrojodid
Bis(m-diethylaminophenolato)bis($\eta^5$-cyclopentadienyl)titan(IV)-dihydrobromid
und
o-Isopropylaminothiophenolato-jodo-bis($\eta^5$-cyclopentadienyl)titan(IV)-dihydrojodid
enthält.

6. Pharmazeutische Zusammensetzung nach Anspruch 4, dadurch gekennzeichnet, daß sie mindestens einen Titanocen-Komplex ausgewählt unter
Bis(hydrogenmaleinato)bis($\eta^5$-cyclopentadienyl)titan(IV)
Bis(trichloracetato)bis($\eta^5$-cyclopentadienyl)titan(IV)
und
Trifluoracetato-fluoro-bis($\eta^5$-cyclopentadienyl)titan(IV)
enthält.

7. Verwendung eines Titanocen-Komplexes der allgemeinen Formel I

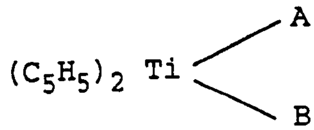

(I)

in der A die Gruppe

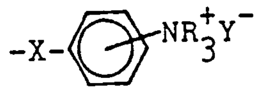

bedeutet, wobei X ein Sauerstoff- oder Schwefelatom ist, R Wasserstoff oder eine $C_{1-4}$-Alkylgruppe ist und Y ein Halogenid ist, oder A die Gruppe —O—CO—R′ bedeutet, wobei R′ die Gruppe $—CF_3$, $—CCl_3$, $—CBr_3$ $—CHCl_2$, $—CH_2Cl$, —CH=CH—COOH oder $—(CH_2)_nCOOH$ ist und n den Wert 0, 1, 2, 3 oder 4 hat, und B ein Halogenid ist oder wie der Rest A definiert ist;
zur Herstellung eines cystostatisch wirksamen Arzneimittels.

8. Verwendung nach Anspruch 7, dadurch gekennzeichnet, daß man einen Titanocen-Komplex ausgewählt unter
Bis(p-aminothiophenolato)bis($\eta^5$-cyclopentadienyl)titan(IV)-dihydrochlorid
p-Aminothiophenolato-chloro-bis($\eta^5$-cyclopentadienyl)titan(IV)-hydrochlorid
Bis-(p-methylaminothiophenolato)bis($\eta^5$-cyclopentadienyl)titan(IV)-dihydrojodid
Bis(p-ethylaminothiophenolato)bis($\eta^5$-cyclopentadienyl)titan(IV)-dihydrojodid
Bis(m-diethylaminophenolato)bis($\eta^5$-cyclopentadienyl)titan(IV)-hydrobromid
und
o-Isopropylaminothiophenolato-jodo-bis($\eta^5$-cyclopentadienyl)titan(IV)-hydrojodid
verwendet.

9. Verwendung nach Anspruch 7, dadurch gekennzeichnet, daß man einen Titanocen-Komplex ausgewählt unter
Bis(hydrogenmaleinato)bis($\eta^5$-cyclopentadienyl)titan(IV)
Bis(trichloracetato)bis($\eta^5$-cyclopentadienyl)titan(IV)
und
Trifluoracetato-fluoro-bis($\eta^5$-cyclopentadienyl)titan(IV)
verwendet.

10. Verfahren zur Herstellung von Titanocen-Komplexe der allgemeinen Formel I

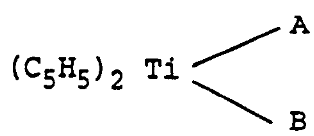

(I)

in der A die Gruppe

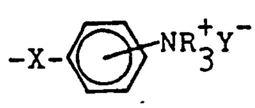

bedeutet, wobei X ein Sauerstoff- oder Schwefelatom ist, R Wasserstoff oder eine $C_{1-4}$-Alkylgruppe bedeutet und Y ein Halogenid ist, und B ein Halogenid ist oder wie der Rest A definiert ist, dadurch gekennzeichnet, daß man ein Titanocen-dihalogenid der allgemeinen Formel II

$$(C_5H_5)_2TiY_2 \qquad (II)$$

in der Y die vorstehende Bedeutung hat,
(a) mit einer Aminophenol- oder Aminothiophenol-Verbindung der allgemeinen Formel III

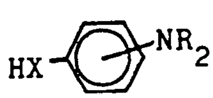

(III)

in der X und R die vorstehende Bedeutung haben zu einem Titanocen-Komplex der allgemeinen Formel I umsetzt oder

(b) mit einem Lithium-aminophenolat oder -aminothiophenolat der allgemeinen Formel IV

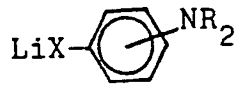

(IV)

in der X und R die vorstehende Bedeutung haben, zu einem Titanocen-Komplex der allgemeinen Formel I'

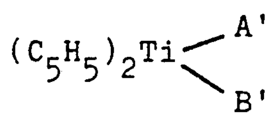

(I')

in der A' die Gruppe

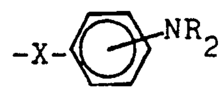

bedeutet und B' ein Halogenid ist oder wie der Rest A' definiert ist, umsetzt und den erhaltenen Titanocen-Komplex der allgemeinen Formel I' durch Umsetzen mit einer Halogenverbindung der allgemeinen Formel V

RY (V)

in der R und Y die vorstehende Bedeutung haben, in einen Titanocen-Komplex der allgemeinen Formel I überführt.

**Revendications**

1. Complexes de titanocènes de formule générale I

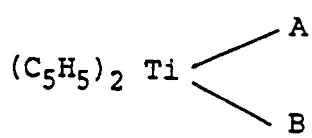

(I)

dans laquelle A représente le groupe

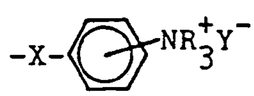

où X représente un atome d'oxygène ou de soufre, R représente un atome d'hydrogène ou un groupe alkyle en $C_1$ à $C_4$, et Y est halogénure, ou bien A représente le groupe —O—CO—R', où R' représente le groupe —$CF_3$, —$CCl_3$, —$CBr_3$ —$CHCl_2$, —$CH_2Cl$, —CH=CH—COOH ou —$(CH_2)_n$COOH, et n vaut 0, 1, 2, 3 ou 4, et B est un halogénure ou a le même sens que celui défini pour le reste A, pour servir de médicamments.

2. Complexes de titanocènes de formule générale I·

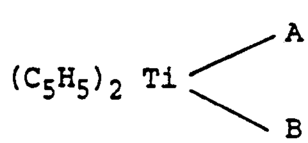

(I)

dans laquelle A représente le groupe

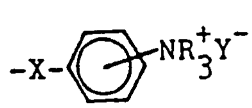

où X représente un atome d'oxygène ou de soufre, R représente un atome d'hydrogène ou un groupe

alkyle en $C_1$ à $C_4$, et Y est un halogénure, et B est un halogénure ou a la même sens que celui défini pour le reste A.

3. Complexes de titanocènes selon la revendication 1, choisis parmi
le dichlorhydrate de bis(p-aminothiophénolato)-bis($\eta^5$-cyclopentadiényl)titane (IV),
le chlorhydrate de p-aminothiophénolato-chloro-bis($\eta^5$-cyclopentadiényl)titane-(IV),
le diiodhydrate de bis-(p-méthylaminothiophénolato)bis($\eta^5$-cyclopentadiényl)titane (IV),
le diiodhydrate de bis(p-éthylaminothiophénolato)bis($\eta^5$-cyclopentadiényl)titane-(IV),
le dibromhydrate de bis(m-diéthylaminophénolato)bis($\eta^5$-cyclopentadiényl)titane-(IV), et
l'iodohydrate d'o-isopropylaminothiophénolato-iodo-bis($\eta^5$-cyclopentadiényl)titane-(IV).

4. Composition pharmaceutique, contenant une quantité efficace d'au moins une complexe de titanocène de formule générale I

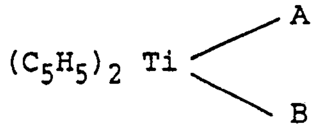

(I)

dans laquelle A représente le groupe

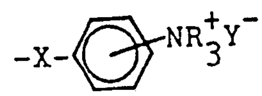

où X représente un atome d'oxygène ou de soufre, R représente un atome d'hydrogène ou un groupe alkyle en $C_1$ à $C_4$, et Y est un halogénure, ou bien A représente le groupe —O—CO—R', où R' représente le groupe —$CF_3$, —$CCl_3$, —$CBr_3$ —$CHCl_2$, —$CH_2Cl$, —CH=CH—COOH ou —$(CH_2)_nCOOH$, et n vaut 0, 1, 2, 3 ou 4, et B est un halogénure ou a le même sens que celui défini pour le reste A;
et des supports, véhicules, excipients ou adjuvants usuels.

5. Composition pharmaceutique selon la revendication 4, caractérisée en ce qu'elle contient au moins un complex de titanocène choisi parmi
le dichlorhydrate de bis(p-aminothiophénolato)-bis($\eta^5$-cyclopentadiényl)titane (IV),
le chlorhydrate de p-aminothiophénolato-chloro-bis($\eta^5$-cyclopentadiényl)titane-(IV),
le diiodhydrate de bis-(p-méthylaminothiophénolato)bis($\eta^5$-cyclopentadiényl)titane (IV),
le diiodhydrate de bis(p-éthylaminothiophénolato)bis($\eta^5$-cyclopentadiényl)titane-(IV),
le dibromhydrate de bis(m-diéthylaminophénolato)bis($\eta^5$-cyclopentadiényl)titane-(IV), et
l'iodhydrate d'o-isopropylaminothiophénolato-iodo-bis($\eta^5$-cyclopentadiényl)titane-(IV).

6. Composition pharmaceutique selon la revendication 4, caractérisée en ce qu'elle contient au moins un complexe de titanocène choisi parmi
le bis(hydrogénomaléinato)bis)$\eta^5$-cyclopentadiényl)-titane-(IV)
le bis(trichloracétato)bis($\eta^5$-cyclopentadiényl)-titane-(IV), et
le trifluoracétato-fluoro-bis($\eta^5$-cyclopentadiényl)-titane-(IV).

7. Utilisation d'un complexe de titanocène de formule générale I

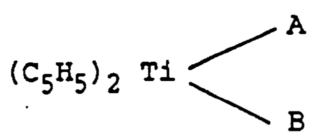

(I)

dans laquelle A représente le groupe

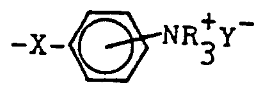

où X représente un atome d'oxygène ou de soufre, R représente un atome d'hydrogène ou un groupe alkyle en $C_1$ à $C_4$, et Y est un halogénure, ou bien A représente le groupe —O—CO—R', où R' représente le groupe —$CF_3$, —$CCl_3$, —$CBr_3$ —$CHCl_2$, —$CH_2Cl$, —CH=CH—COOH ou —$(CH_2)_nCOOH$, et n vaut 0, 1, 2, 3 ou 4, et B est un halogénure ou a le même sens que celui défini pour le reste A;
pour préparer un médicament à action cytostatique.

8. Utilisation selon la revendication 7, caractérisée en ce qu'on utilise un complexe de titanocène choisi parmi
le dichlorhydrate de bis(p-aminothiophénolato)-bis($\eta^5$-cyclopentadiényl)titane (IV),

le chlorhydrate de aminothiophénolato-chloro-bis($\eta^5$-cyclopentadiényl)titane-(IV),
le diiodhydrate de bis-(p-méthylaminothiophénolato)bis($\eta^5$-cyclopentadiényl)titane (IV),
le diiodhydrate de bis(p-éthylaminothiophénolato)bis($\eta^5$-cyclopentadiényl)titane-(IV),
le dibromhydrate de bis(m-diéthylaminophénolato)bis($\eta^5$-cyclopentadiényl)titane-(IV), et
l'iodhydrate d'o-isopropylaminothiophénolato-iodo-bis($\eta^5$-cyclopentadiényl)titane-(IV).

9. Utilisation selon la revendication 7, caractérisée en ce qu'on utilise un complexe de titanocène, choisi parmi
le bis(hydrogénomaléinato)bis)$\eta^5$-cyclopentadiényl)-titane-(IV),
le bis(trichloracétato)bis($\eta^5$-cyclopentadiényl)-titane-(IV), et
le trifluoracétato-fluoro-bis($\eta^5$-cyclopentadiényl)-titane-(IV).

10. Procédé pour préparer des complexes de titanocènes de formule générale I

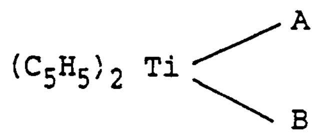

(I)

dans laquelle A représente le groupe

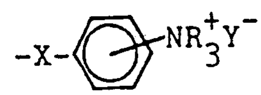

où X représente un atome d'oxygène ou de soufre, R représente un atome d'hydrogène ou un groupe alkyle en $C_1$ à $C_4$ et Y est un halogénure et B est un halogénure ou a la même sens ce que celui défini pour le reste A, procédé caractérisé en ce qu'on fait réagir un dihalogénure de titanocène de formule générale II:

$$(C_5H_5)_2TiY_2 \qquad (II)$$

dans laquelle Y a le sens précité:
(a) avec un aminophénol ou avec un aminothiophénol de formule générale (III):

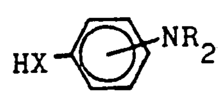

(III)

(dans laquelle X et R ont le sens précité) pour obtenir un complexe de titanocène de formule générale I, ou
(b) avec un aminophénolate ou un aminothiophénolate de lithium de formule générale IV

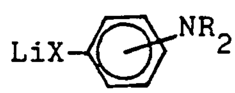

(IV)

(dans laquelle X et R ont le sens précité) pour obtenir un complexe de titanocène de formule générale I'

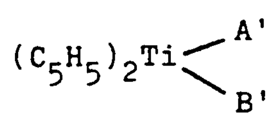

(I')

dans laquelle A' représente le groupe

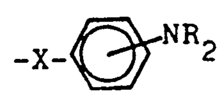

et B' est un halogénure ou a le même sens que celui défini pour le reste A', et, par réaction avec un halogénure de formule générale V:

$$RY \qquad (V)$$

(dans laquelle R et Y ont le sens précité), ou transforme le complexe de titanocène de formule générale I', ainsi obtenu, en un complexe de titanocène de formule générale I.

**Claims**

1. Titanocene complexes having the general formula I

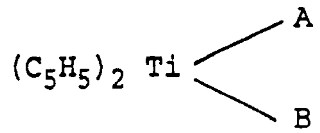

(I)

wherein A represents the group

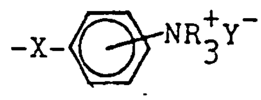

in which X is an oxygen atom or sulfur atom, R is hydrogen or a $C_{1-4}$-alkyl group, and Y is a halide; or A represents the group —O—CO—R', in which R' is the group $—CF_3$, $—CCl_3$, $—CBr_3$, $—CHCl_2$, $—CH_2Cl$, —CH=CH—COOH or $—(CH_2)_nCOOH$, and n has the value 0, 1, 2, 3 or 4, and B is a halide or is defined as the radical A;
for use as pharmaceuticals.

2. Titanocene complexes having the general formula I

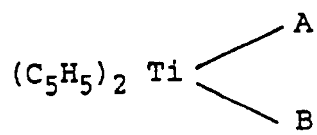

(I)

wherein A represents the group

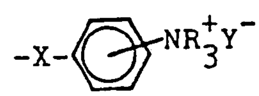

in which X is an oxygen atom or sulfur atom, R is hydrogen or a $C_{1-4}$-alkyl group, and Y is a halide, and B is a halide or is defined as the radical A.

3. Titanocene complexes according to claim 1 selected from:
bis(p-amino thiophenolato)bis($\eta^5$-cyclopentadienyl)titanium(IV) dihydrochloride,
p-amino thiophenolato-chloro-bis($\eta^5$-cyclopentadienyl)titanium(IV) hydrochloride,
bis-(p-methylaminothiophenolato)bis($\eta^5$-cyclopentadienyl)titanium(IV) dihydroiodide,
bis(p-ethylaminothiophenolato)bis($\eta^5$-cyclopentadienyl)titanium(IV) dihydroiodide,
bis(m-diethylaminophenolato)bis($\eta^5$-cyclopentadienyl)titanium(IV) hydrobromide
and
o-isopropylaminothiophenolato-iodo-bis($\eta^5$-cyclopentadienyl)titanium(IV) hydroiodide.

4. Pharmaceutical composition comprising an effective amount of at least one titanocene complex having the general formula I

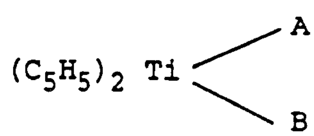

(I)

wherein A represents the group

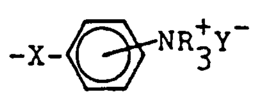

in which X is an oxygen atom or sulfur atom, R is hydrogen or a $C_{1-4}$-alkyl group, and Y is a halide; or A represents the group —O—CO—R', in which R' is the group $—CF_3$, $—CCl_3$, $—CBr_3$, $—CHCl_2$, $—CH_2Cl$, —CH=CH—COOH or $—(CH_2)_nCOOH$, and n has the value 0, 1, 2, 3 or 4, and B is a halide or is defined as the radical A;
and conventional carriers or excipients.

5. Pharmaceutical composition according to claim 4, characterized in that it comprises at least one titanocene complex selected from
bis(p-aminothiophenolato)bis($\eta^5$-cyclopentadienyl)titanium(IV) dihydrochloride,
p-aminothiophenolato-chloro-bis($\eta^5$-cyclopentadienyl)titanium(IV) hydrochloride,
bis-(p-methylaminothiophenolato)bis($\eta^5$-cyclopentadienyl)titanium(IV) dihydroiodide,
bis(p-ethylaminothiophenolato)bis($\eta^5$-cyclopentadienyl)titanium(IV) dihydroiodide,
bis(m-diethylaminophenolato)bis($\eta^5$-cyclopentadienyl)titanium(IV) dihydrobromide
and
o-isopropylaminothiophenolato-iodo-bis($\eta^5$-cyclopentadienyl)titanium(IV) hydroiodide.

6. Pharmaceutical composition according to claim 4, characterized in that it contains at least one titanocene complex selected from
bis(hydrogenmaleinato)bis($\eta^5$-cyclopentadienyl)titanium(IV),
bis(trichloroacetato)bis($\eta^5$-cyclopentadienyl)titanium(IV)
and
(trifluoroacetato-fluoro-bis($\eta^5$-cyclopentadienyl)titanium(IV).

7. The use of a titanocene complex having the general formula I

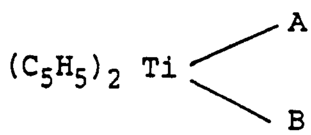

(I)

wherein A represents the group

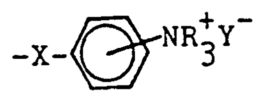

in which X is an oxygen atom or sulfur atom, R is hydrogen or a $C_{1-4}$-alkyl group, and Y is a halide; or A represents the group —O—CO—R', in which R' is the group $—CF_3$, $—CCl_3$, $—CBr_3$, $—CHCl_2$, $—CH_2Cl$, —CH=CH—COOH or $—(CH_2)_nCOOH$, and n has the value 0, 1, 2, 3 or 4, and B is a halide or is defined as the radical A;
for the preparation of a cytostatically effective pharmaceutical.

8. The use according to claim 7, characterized in that a titanocene complex is used which is selected from
bis(p-aminothiophenolato)bis($\eta^5$-cyclopentadienyl)titanium(IV) dihydrochloride,
p-aminothiophenolato-chloro-bis($\eta^5$-cyclopentadienyl)titanium(IV) hydrochloride,
bis-(p-methylaminothiophenolato)bis($\eta^5$-cyclopentadienyl)titanium(IV) dihydroiodide,
bis(p-ethylaminothiophenolato)bis($\eta^5$-cyclopentadienyl)titanium(IV) dihydroiodide,
bis(m-diethylaminophenolato)bis($\eta^5$-cyclopentadienyl)titanium(IV) hydrobromide
and
o-isopropylaminothiophenolato-iodo-bis($\eta^5$-cyclopentadienyl)titanium(IV) hydroiodide.

9. The use according to claim 7, characterized in that a titanocene complex is used which is selected from
bis(hydrogenmaleinato)bis($\eta^5$-cyclopentadienyl)titanium(IV),
bis(trichloroacetato)bis($\eta^5$-cyclopentadienyl)titanium(IV)
and
(trifluoroacetato-fluoro-bis($\eta^5$-cyclopentadienyl)titanium(IV).

10. A process of the preparation of titanocene complexes having the general formula I

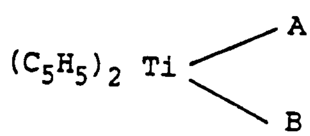

(I)

wherein A represents the group

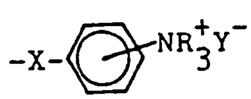

in which X is an oxygen atom or sulfur atom, R is hydrogen or a $C_{1-4}$-alkyl group, and Y is a halide, and B is a halide or is defined as the radical A, characterized in that a titanocene dihalide having the general formula II

$$(C_5H_5)_2TiY_2 \qquad (II)$$

wherein Y has the meaning as defined above,

(a) is reacted with an aminophenol or amino thiophenol of the general formula III

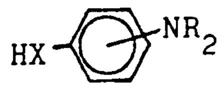

(III)

wherein X and R have the meaning as defined above, to form a titanocene complex of the general formula I or

(b) is reacted with a lithium aminophenolate or amino thiophenolate of the general formula IV

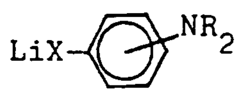

(IV)

wherein X and R have the meaning as defined above, to form a titanocene complex of the general formula I'

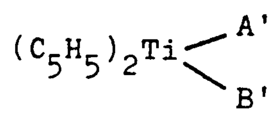

(I')

wherein A' represents the group

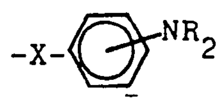

and B' is a halide or is defined as the radical A', and the thus obtained titanocene complex having general formula I' is converted into a titanocene complex of the general formula I by reaction with a halide compound having the general formula V

$$RY \qquad (V)$$

wherein R and Y have the meaning as defined above.

IN-VIVO-BEHANDLUNG VON EHRLICH-ASZITES-TUMOR

FIG. 1

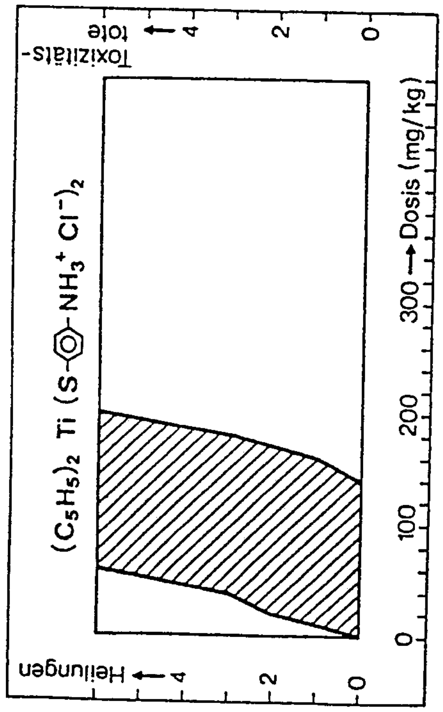


FIG. 2

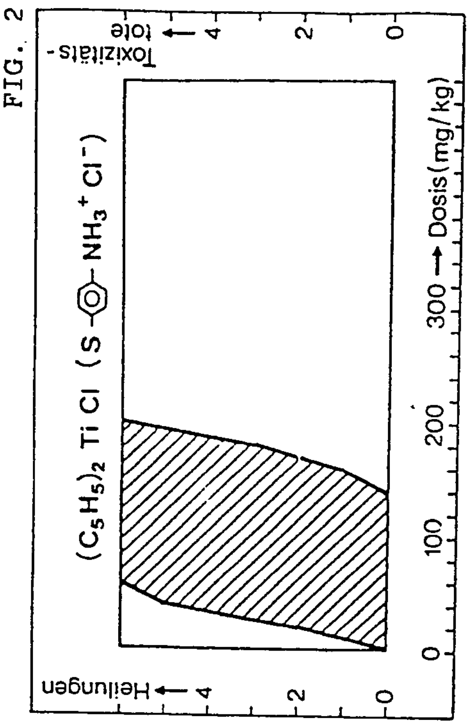


FIG. 3

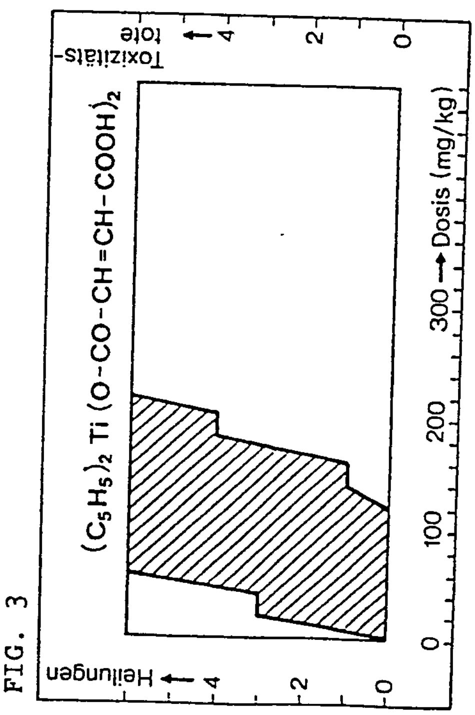


FIG. 4

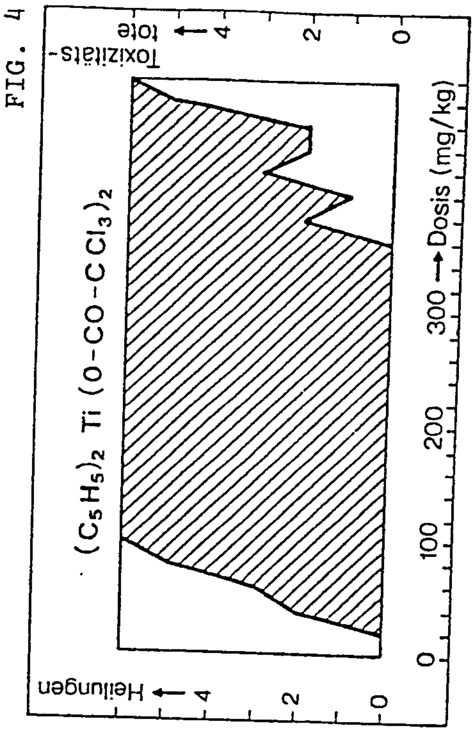

FIG. 5

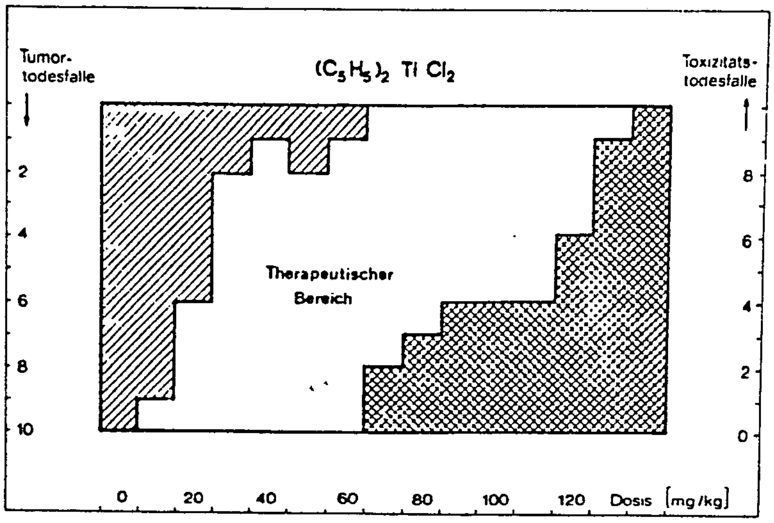
Tumor-
todesfalle
$(C_5H_5)_2$ Ti $Cl_2$
Toxizitats-
todesfalle
Therapeutischer
Bereich
2
4
6
8
10
8
6
4
2
0
0
20
40
60
80
100
120
Dosis [mg/kg]

Human-Adenocarcinom der Lunge

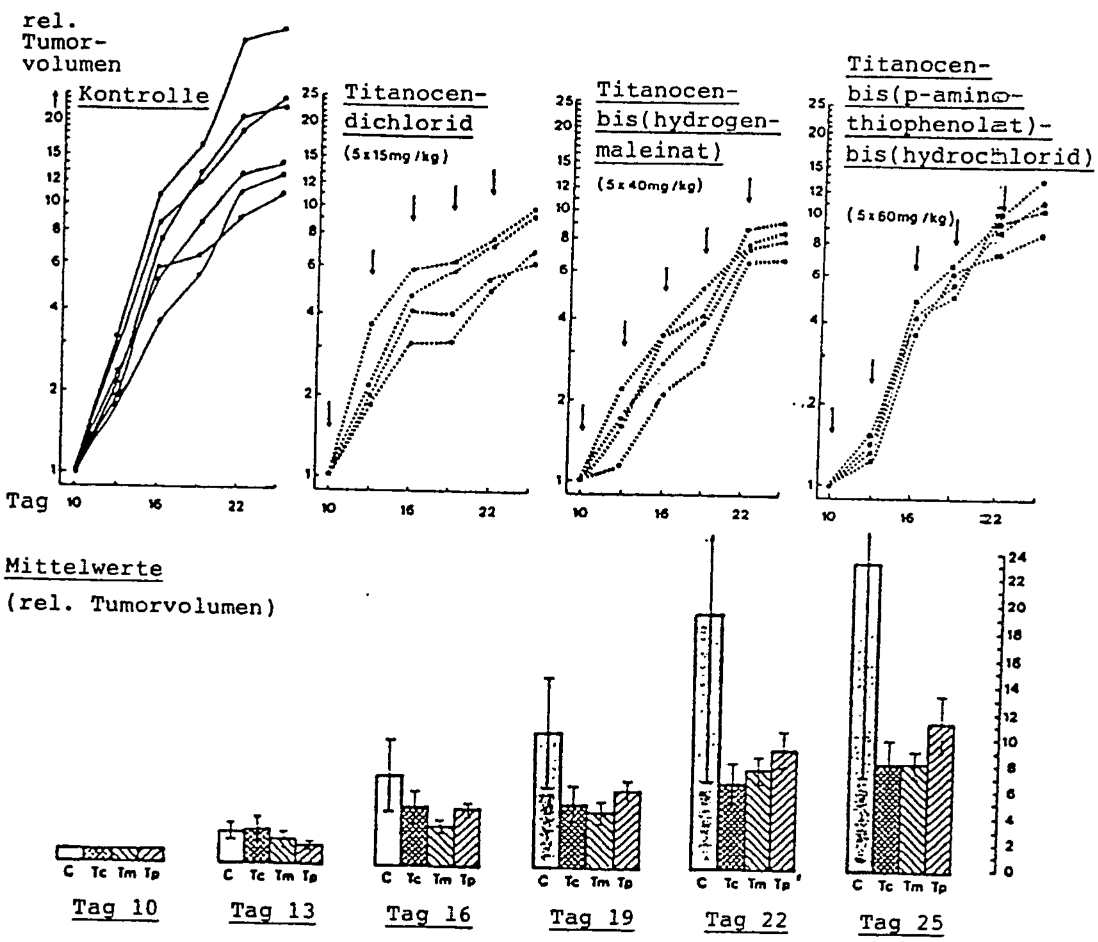


C = Kontrolle Tc = Titanocen-dichlorid Tm = Titanocen-bis(hydrogen-maleinat)
Tp = Titanocen-bis(p-aminothiophenolat)-bis(hydrochlorid)

FIG. 6

Human-Adenocarcinom des Rektums

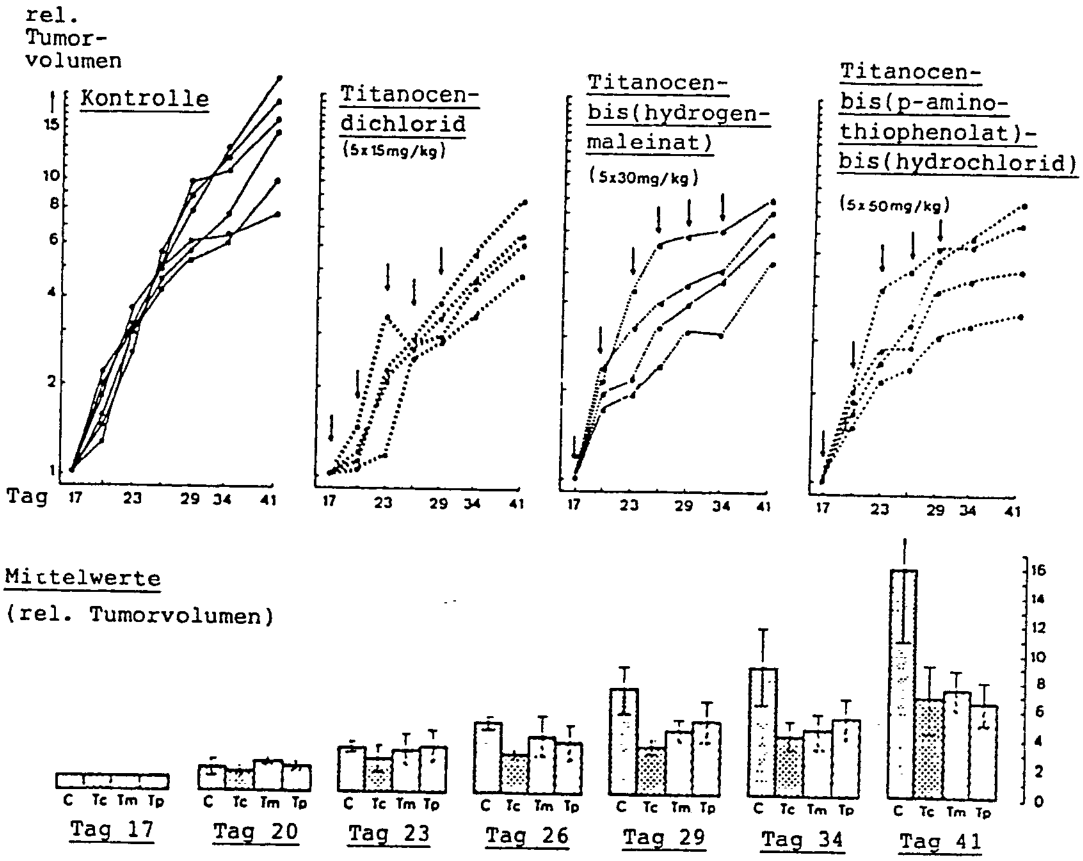


C = Kontrolle Tc = Titanocen-dichlorid Tm = Titanocen-bis(hydrogen-maleinat)

Tp = Titanocen-bis(p-aminothiophenolat)-bis(hydrochlorid)

FIG. 7